# EUROPEAN PATENT SPECIFICATION

(11) **EP 4 035 669 B1**
(45) Date of publication and mention of the grant of the patent: **08.10.2025**
(21) Application number: 20871356.0
(22) Date of filing: 30.09.2020
(51) Int. Cl.: A61K 31/522, A61K 31/573, A61P 25/28, A61P 25/00, A61K 9/16, A61K 9/20, A61K 9/48

(54) **COMBINATION OF ACYCLOVIR AND DEXAMETHASONE FOR USE IN TREATING ALZHEIMER'S DISEASE**
KOMBINATION AUS ACICLOVIR UND DEXAMETHASON ZUR VERWENDUNG IN DER BEHANDLUNG DER ALZHEIMER-KRANKHEIT
COMBINAISON D'ACYCLOVIR ET DE DEXAMÉTHASONE POUR SON UTILISATION DANS LE TRAITEMENT DE LA MALADIE D'ALZHEIMER

(30) Priority: 30.09.2019 CN 201910942002
(43) Date of publication of application: 03.08.2022
(73) Proprietor: Plantarx Limited, Hong Kong SAR (HK)
(72) Inventor: CHOI, Tony Chunglit, Hong Kong SAR, China Hong Kong 0 (CN)
(74) Representative: Chung, Hoi Kan
(86) International application number: PCT/CN2020/119593
(87) International publication number: WO 2021/063408

(56) References cited:
- WO-A2-2005/019166
- WO-A2-2012/048330
- CN-A- 1 179 718
- CN-A- 1 981 771
- CN-A- 101 342 180
- CN-A- 101 940 789
- CN-A- 103 948 605
- CN-A- 109 045 043
- CHEN ZHAOHONG ET AL: "Effect of Dexamethasone and Acyclovir on Prognosis and Expression of IL-2 in Mice with Herpes Simplex Virus Encephalitis", LIN CHUANG ER KE ZAZHI:SHUANG YUE KAN = THE JOURNAL OF CLINICAL PEDIATRICS : JCP, vol. 30, no. 11, 1 November 2012 (2012-11-01), CN, pages 1063 - 1066, XP055797366, ISSN: 1000-3606, DOI: 10.3969/j.issn.1000-3606.2012.11.018
- CUMMINGS JEFFREY ET AL: "Alzheimer's disease drug development pipeline: 2021", vol. 7, no. 1, 1 January 2021 (2021-01-01), XP055969848, ISSN: 2352-8737, Retrieved from the Internet <URL:https://onlinelibrary.wiley.com/doi/full-xml/10.1002/trc2.12179> DOI: 10.1002/trc2.12179
- DEVANAND D P ET AL: "Antiviral therapy: Valacyclovir Treatment of Alzheimer's Disease (VALAD) Trial: protocol for a randomised, double-blind,placebo-controlled, treatment trial", vol. 10, no. 2, 1 February 2020 (2020-02-01), London, UK, pages e032112, XP055969544, ISSN: 2044-6055, Retrieved from the Internet <URL:https://bmjopen.bmj.com/content/bmjopen/10/2/e032112.full.pdf> DOI: 10.1136/bmjopen-2019-032112
- HUI ZHANG; ZHIJUN YUAN; YUSHAN YAN; LIPING CHEN; YIYING ZHOU; DIFAN ZHANG; CHUNGLIT CHOI TONY; WEI CUI: "The combination of acyclovir and dexamethasone protects against Alzheimer’s disease-related cognitive impairments in mice", PSYCHOPHARMACOLOGY., SPRINGER VERLAG, BERLIN., DE, vol. 237, no. 6, 27 March 2020 (2020-03-27), DE, pages 1851 - 1860, XP037143529, ISSN: 0033-3158, DOI: 10.1007/s00213-020-05503-1
- ITZHAKI RUTH F.: "Herpes simplex virus type 1 and Alzheimerâ€™s disease: increasing evidence for a major role of the virus", FRONTIERS IN AGING NEUROSCIENCE, vol. 6, 11 August 2014 (2014-08-11), pages 1 - 9, XP055797356, DOI: 10.3389/fnagi.2014.00202
- JOSHI YASH B., CHU JIN, PRATICÒ DOMENICO: "Stress Hormone Leads to Memory Deficits and Altered Tau Phosphorylation in a Model of Alzheimer's Disease", JOURNAL OF ALZHEIMER'S DISEASE, IOS PRESS, NL, vol. 31, no. 1, 5 July 2012 (2012-07-05), NL, pages 167 - 176, XP055797357, ISSN: 1387-2877, DOI: 10.3233/JAD-2012-120328
- WEI, HENGYANG ET AL.: "Acyclovir Combined with Dexamethasone for the Treatment of Herpes Simplexe Virus Encephalitis in Mice", CHINESE JOURNAL OF EXPERIMENTAL AND CLINICAL VIROLOGY, vol. 28, no. 6, 31 December 2014 (2014-12-31), pages 443 - 445, XP009527076, ISSN: 1003-9279
- HARRIS STEVEN A., HARRIS ELIZABETH A.: "Molecular Mechanisms for Herpes Simplex Virus Type 1 Pathogenesis in Alzheimer’s Disease", FRONTIERS IN AGING NEUROSCIENCE, vol. 10, 6 March 2018 (2018-03-06), XP055797359, DOI: 10.3389/fnagi.2018.00048
- WOZNIAK M.A.; FROST A.L.; ITZHAKI R.F.: "The helicase-primase inhibitor BAY 57–1293 reduces the Alzheimer’s disease-related molecules induced by herpes simplex viru", ANTIVIRAL RESEARCH, ELSEVIER BV, NL, vol. 99, no. 3, 16 July 2013 (2013-07-16), NL, pages 401 - 404, XP028728470, ISSN: 0166-3542, DOI: 10.1016/j.antiviral.2013.07.003
- CHEN ZHAOHONG, HUA QING, SHU ZHI-RONG, ZHAO YUAN-YUAN, MA KAI, WANG TIAN-TIAN, XU YI-FENG: "Effect of Dexamethasone and Acyclovir on Prognosis and Expression of IL-2 in Mice with Herpes Simplex Virus Encephalitis", JOURNAL OF CLINICAL PEDIATRICS, vol. 30, no. 11, 1 November 2012 (2012-11-01), pages 1063 - 1066, XP055797366, DOI: 10.3969 j.issn.1000-3606.2012.11.018

## Description

### TECHNICAL FIELD

The present disclosure relates to new uses of drugs, in particular a combination of acyclovir and dexamethasone (DXMT) for use in treating Alzheimer's disease (AD).

### BACKGROUND ART

Alzheimer's disease (AD) is the most common chronic and irreversible neurodegenerative disease in world. With an increasingly large aging population, AD becomes one of the greatest medical challenges. However, effective drugs for this disease have not yet been developed.. Cognitive impairment in AD is associated with cholinergic degeneration and excitotoxicity. Therefore, cholinesterase inhibitors including donepezil, rivastigmine, and galantamine, and an N-methyl-D-aspartate (NMDA) receptor antagonist memantine, are used clinically to treat AD. However, these drugs only relieve symptoms, and cannot prevent or delay progression of AD.

Acyclovir (guanosine analog) is used as an antiviral drug for a treatment of herpes simplex encephalitis. Previous studies have shown that acyclovir could inhibit tryptophan-2,3-dioxygenase activity both *in vitro* and *in vivo,* leading to increases of serotonin and 5-hydroxyindole acetic acid levels¹. The acyclovir has been proved to be capable of reducing clinical symptoms of depression in patients with the herpes simplex encephalitis. In addition, the acyclovir has been reported to be associated with mortality from the AD, but it is unknown whether the acyclovir is neuroprotective².

Dexamethasone (DXMT), a synthetic glucocorticoid, is clinically used for treatment of allergic reactions and inflammatory skin conditions³. DXMT has a wide range of functions in central nervous, such as regulation of gene transcription and modulation of synaptic structures⁴. Long-term treatment with the DXMT can induce pathological changes of tau and enhance expression of Aβ; however, it is unknown whether the DXMT can improve cognitive impairments. However, short-term treatment with the DXMT suppresses AD-related neuroinflammation and prevents the cognitive impairment⁴. Interestingly, maternal DXMT exposure could ameliorate cognitive impairments in an offspring of AD transgenic mice, suggesting beneficial effects of DXMT for AD prevention⁴.

Recently, co-application of acyclovir and DXMT in an early stage of herpes simplex encephalitis can effectively alleviate the neurological symptoms, and improve survival rate in mice, indicating usefulness of acyclovir and DXMT combination in treatment of neurological disease⁶. However, whether acyclovir and DXMT combination could protect against AD-related cognitive impairments are unclear in a large extent.

ITZHAKI, RF. Frontiers in Aging Neuroscience, vol. 6, August 2014, pages 1-9, XP055797356, DOI: 10.3389/fnagi.2014.00202 concludes for the presence of HSV1 in the elderly human brain, for its reactivation there, its interference with cellular processes and its harmful effects on cognition, is very strong.

In the present disclosure, the combination therapy with the acyclovir and the DXMT is evaluated on a treatment of neuroinflammation due to AD-induced Aβ oligomers, and protective effects of synaptic protein expression and cognition in mice.

In the present study, it is found for the first time that a combination, rather than single use, of the acyclovir and the DXMT, can prevent spatial cognitive impairment caused by β-amyloid (Aβ) oligomers in AD. In addition, the acyclovir and the DXMT can also prevent Aβ oligomer-induced hyperactivation of microglia and astrocytes, and overexpression of pro-inflammatory cytokines. This suggests that an anti-AD effect of a drug combination therapy may be achieved, at least partially, through inhibition of neuroinflammation and immunoregulation effects. Moreover, combined use of the acyclovir and the DXMT can block a Aβ oligomer-induced decrease of PSD95 expression, suggesting a protective effect of a drug combination on synapses.

Therefore, the present disclosure provides a method for use in treating AD with a combination of drugs.

### SUMMARY

The present disclosure provide acyclovir and DXMT for use in treating AD.

The present disclosure has found that:
DXMT ameliorates acyclovir-induced weight loss in mice with Aβ oligomers.

Combination of the acyclovir and the DXMT can significantly alleviate Aβ oligomer-induced spatial cognitive impairment.

Combination of the acyclovir and the DXMT can significantly reduce Aβ oligomer-induced overexpression of pro-inflammatory cytokines.

Combination of the acyclovir and the DXMT can significantly reduce Aβ oligomer-induced activation of microglia and astrocytes.

Combination of the acyclovir and the DXMT can significantly reduce Aβ-induced decrease in PSD-95 expression.

Combination of the acyclovir and the DXMT can significantly reduce Aβ oligomer-induced pTau expression.

The above findings suggest that the present disclosure has developed a new alternative therapy for AD, namely use of a combination of acyclovir and DXMT in preparation of a drug for treating AD.

A use of the present disclosure includes all symptoms of a patient with the AD, especially following symptoms: cognitive impairment and neuroinflammation.

It is further found in the present disclosure that combination of acyclovir and DXMT has a synergistic effect. The DXMT can improve acyclovir-induced weight loss while can perform anti-inflammatory and immunoregulation effects.

In the present disclosure, the acyclovir and pharmaceutically acceptable salts thereof can be used in any one of pharmaceutically acceptable forms, preferably in oral preparations, such as tablets, capsules, and granules.

In the present disclosure, the DXMT and a pharmaceutically acceptable salt thereof, such as DXMT acetate and DXMT sodium phosphate, can be used in any one of pharmaceutically acceptable forms, preferably in oral preparations, sucn as tablets, capsules, and granules.

In the present disclosure, the acyclovir and the DXMT are used simultaneously or successively, with an effective dosage; for example, acyclovir 500 mg per time and DXMT 1.5 mg per time are used both twice a day; each gram of a cream in a medicinal cold cream base should include 20 mg of acyclovir and 0.25 mg of DXMT for acting simultaneously *in vivo.* If necessary, according to a difference of drug metabolism, a drug with slow metabolism can be administered, followed by a drug with fast metabolism after the drug with slow metabolism is absorbed to simultaneously act *in vivo.* Accordingly, the present disclosure includes a combination package, including an acyclovir preparation in one package and a DXMT preparation in another package.

In the present disclosure, the combination further includes preparing the acyclovir and the DXMT into a compound pharmaceutical composition; that is, putting two drugs into one preparation; where the two drugs are taken at the same time when taking the preparation.

For the compound pharmaceutical composition, a weight ratio of the two drugs needs to be specified at (3000-200):(10-0.1), preferably 500:(1-5), most preferably 500:(1.5-2).

The compound pharmaceutical composition can be taken into the human body by any route of administration, including gastrointestinal administration, intravenous administration, intramuscular injection, subcutaneous injection, and oral or nasal mucosa administration, preferably by the gastrointestinal administration. In the present disclosure, the compound pharmaceutical composition can be prepared into any ingestible dosage form, preferably an oral preparation; the compound pharmaceutical composition can be added with a pharmaceutically acceptable carrier if necessary, for example, active pharmaceutical ingredients in the preparation may have a weight percentage of 0.1% to 99.9%, and a pharmaceutically acceptable carrier as a balance.

In the present disclosure, an oral preparation can be any pharmaceutically acceptable dosage forms, and these dosage forms include: tablets, capsules, oral liquids, buccal preparations, granules, dissolved medicines, pills, powders, suspensions, powders, drops, dropping pills, microspheres and sprays. The preparation is preferably an oral dosage form, including: the capsules, the tablets, the oral liquids, the granules, and sprays.

In the present disclosure, the oral preparation may include commonly used excipients, including binders, fillers, diluents, tablets, lubricants, disintegrating agents, coloring agents, flavoring agents and wetting agents; and if necessary, the tablets may be coated.

In the present disclosure, each dosage of the oral preparation may include 500 mg of the acyclovir and 1.5 mg of the DXMT, taken twice a day.

In the present disclosure, each dosage of the oral preparation may include 500 mg of the acyclovir and 2.0 mg of the DXMT, taken twice a day.

A suitable filler includes cellulose, mannitol, lactose and other similar fillers. A suitable disintegrating agent includes starch, polyvinylpyrrolidone, and a starch including sodium starch glycolate. A suitable lubricant includes magnesium stearate. A suitable pharmaceutically acceptable humectant includes sodium lauryl sulfate. A solid oral composition can be prepared by conventional methods including mixing, filling, and tabletting. Repeated mixing allows active substances to be distributed throughout those compositions where a large amount of fillers are used.

An oral liquid preparation may be in a form including aqueous or oily suspensions, solutions, emulsions, syrups or elixirs, or may be a dry product for reconstituting with water or other suitable carriers before use. Such liquid preparations may include a conventional additive including a suspending agent, such as sorbitol, syrup, methylcellulose, gelatin, hydroxyethylcellulose, carboxymethylcellulose, aluminum stearate gel or hydrogenated edible fats, and an emulsifier, such as lecithin, sorbitan monooleate and acacia; a non-aqueous carrier (which may include edible oils) includes almond oil, fractionated coconut oil, oily esters such as glycerol esters, propylene glycol, and ethanol; a preservative includes methylparaben or propylparaben, and sorbic acid; in addition, if desired, conventional flavouring agents or colouring agents may be used.

In the present disclosure, when the oral preparation is prepared into a drug, a suitable pharmaceutically acceptable carrier can be optionally added, including: mannitol, sorbitol, sodium metabisulfite, sodium bisulfite, sodium thiosulfate, cysteine hydrochloride, thioglycolic acid, methionine, vitamin C, disodium EDTA, calcium sodium EDTA, monovalent alkali metal carbonate, acetate, phosphate or a aqueous solution thereof, hydrochloric acid, acetic acid, sulfuric acid, phosphoric acid, amino acid, sodium chloride, potassium chloride, sodium lactate, xylitol, maltose, glucose, fructose, dextran, glycine, starch, sucrose, lactose, mannitol, silicon, cellulose, alginate, gelatin, polyvinylpyrrolidone, glycerin, Tween-80, agar, calcium carbonate, calcium bicarbonate, surfactants, polyethylene glycol, cyclodextrin, β-cyclodextrin, phospholipid materials, kaolin, talc, calcium stearate, and magnesium stearate.

In the present disclosure, when being prepared into a pharmaceutical preparation, the compound pharmaceutical composition can be prepared by conventional techniques of pharmaceutical engineering; for example, active ingredients are used as a raw material, a certain amount of pharmaceutically acceptable carriers is added, followed by mixing evenly, and tablets, capsules, and granules can be prepared according to characteristics of a dosage form.

In the present disclosure, the drug is a balance regulator of an immune system, and relieves brain inflammation and autoimmune hyperfunction; therefore, the drug has a certain therapeutic resistance to invasion of the brain by protozoa, bacteria, viruses, fungi, and parasites and has a certain ability to protect and repair endogenous nerves, blood vessels and cranium.

In the present disclosure, the drug is capable of being combined with a target therapy for senile dementia and the AD based on immune balance, to achieve synergistic treatment and repair, and synergistic treatment and repair through natural regulation of immune system cells and factors, thereby shortening a time required for treatment with more safety and less sequelae.

In the present disclosure, the drug is used in pregnant women, newborn infants, infants and children, adults, and the elderly, especially in fatal brain infections caused by a human herpes simplex virus and near-derived varicella virus infections, with post-treatment immune memory protection.

The functions and effects of the present disclosure are further described below through animal experiments.

### Materials and methods

### Chemicals and reagents

Acyclovir is supplied by Sichuan Collen Pharmaceutical Co., Ltd. DXMT is produced by Zhejiang Xianju Pharmaceutical Co., Ltd. Donepezil is produced by Santa Cruz Biotechnology (Shanghai, China). Aβ1-42 peptide is synthesized by GL Biochem (Shanghai, China).

### Preparation of an Aβ oligomer

An Aβ oligomer⁷ is obtained as previously described. Briefly, Aβ is added in hexafluoroisopropanol (HFIP, Sigma, St. Louis, Mo, USA) to form an Aβ monomer. The Aβ monomer is treated in a 10% hexafluoroisopropanol solution under spin vacuum. The hexafluoroisopropanol is evaporated to obtain an Aβ solution. The Aβ solution is incubated at 25°C for 2 d with agitation, and centrifuged at 14,000 g for 15 min at 4°C, and a resulting supernatant mainly includes a soluble Aβ oligomer. The supernatant is analyzed by BCA (collected and quantified).

### Animal research and drug therapy

The trial has followed guidelines of the Animal Research Advisory Committee of Ningbo University on use and care of animals. Zhejiang Academy of Medical Sciences has provided ICR mice weighing about 25 g. The mice are subjected to a 12-h illumination/darkness cycle at 22±2°C (humidity: 50±10%), and are provided with water and standard food. The acyclovir, DXMT and donepezil were dissolved in water. The mice are randomly divided into 6 groups with 10 mice in each group, including: a control group, an Aβ oligomer group, an Aβ+500 mg/kg acyclovir group, an Aβ+2 mg/kg DXMT group, an Aβ+500 mg/kg acyclovir, 2 mg/kg DXMT group, and an Aβ+2.5 mg/kg donepezil group. The mice are anesthetized by intraperitoneal injection of sodium pentobarbital (50 mg/kg), and placed in a stereotaxic apparatus (RWD Life Science Co., Ltd., Shenzhen, China).

On day 1, the Aβ oligomer was injected into a bilateral ventricle region. A micro-dose is injected (1 µl/side) over 5 min. To reduce backflow, the cannula is left in place for 5 min. Previous study has found that the Aβ oligomers in a brain cavity of mice injected with ICR may lead to cognitive impairment^{7,8}. Mice recover for 3 d without any experiments. On a 4th day, the mice in the experimental group and the positive control group were gavaged for 13 d (twice a day, with 0.2 ml per day). The control group is given a normal saline for 13 d.

### Open field experiment

To analyze a motor activity and exploratory behavior, the mice are housed in an open field (50 cm × 50 cm × 39 cm) with white plywood walls and brown floors. The open field is divided into four equal-length squares (25 cm × 25 cm). The mice are placed one by one in a center of a box, and allowed to fumble for 5 min. The number of crossed lines of four paws and the number of feedings are recorded using a hand-held counter and stopwatch, which are used to mark the motor activity and exploratory behavior. Single-blind monitoring is conducted on drug effects in the mice by an independent researcher. To avoid disturbance of the animals by urine and feces, the open field is cleaned with a 10% alcohol solution and a dry towel between the two trials.

### Morris water maze test

The spatial memory is assessed with the Morris water maze test¹⁰. The Morris water maze is a circular pool (110 cm in diameter) filled with water at a temperature of 23±2°C, and has a platform. Except on a last day, the platform is always placed in a center of a northwest quadrant. Swimming tracks are recorded by a video recorder connected to a computer. Each mouse is trained on positioning the platform over a four-day trial, and learning is assessed daily for four consecutive days. The time mice take to enter a hidden platform is measured. On the last day, the platform is removed and the mice are trained to swim for 90 sec for a probe trial. The swimming times are recorded in the four quadrants of the pool. A tendency to appear in the quadrant occupied before the platform indicates a spatial memory ability.

### Brain tissue collection

One day after the Morris water maze test, mice are deeply anesthetized and transcardially perfused with an ice-cold saline. The brain is quickly dissected. Hippocampal proteins are extracted and stored at -80°C prior to use in a Western blotting assay (4 mice per group) and an enzyme-linked immunosorbent assay (ELISA, 3 mice per group). Intact brain tissues (3 mice per group) are stored in a formalin solution at -80°C prior to staining using immunohistochemistry (IHC).

### ELISA assay

Determination was conducted on concentrations of interleukin-6 (IL-6) and tumor necrosis factor-α (TNF-α) in mouse hippocampus using freshly prepared brain tissues. Brain samples are homogenized in a 0.1M phosphate buffered saline. A resulting brain extract is centrifuged to 2,000 g at 4°C for 15 min. Concentrations of IL-6 and TNF-α are determined with an ELISA kit (Excell Bio, Shanghai, China) according to the manufacturer's protocol. An absorbance is read.

### Western blotting assay

The Western blotting assay is conducted as previously described¹¹. Briefly, the brain tissues from hippocampus are extracted in a lysis buffer for 1 min and centrifuged at 16,000 g for 10 min. Protein levels in a supernatant are estimated by a Bradford analysis, the proteins are separated on an SDS-polyacrylamide gel, and electrotransferred to a polyvinylidene fluoride membrane. After 2 h of incubation with 5% skim milk in TBST, incubation is conducted for 12 h with corresponding antibodies Tau, IL-17 (Santa Cruz Biotechnology, Shanghai, China), pSer396-Tau, PSD-95, and β-actin (Cell Signaling Technology, Inc., Beverly, MA, USA). After washing the samples three times with the TBST, a membrane is incubated with a secondary antibody. Subsequently, the membrane is developed using a chemiluminescence combination kit (Amersham Biotechnology Co., Ltd., Aylesbury, UK), and the signal is exposed to an autoradiographic film. All experimental data is representative of three independent experiments. The data is expressed as a ratio of an optical density (OD) compared to the control and a statistical analysis.

### IHC staining

IHC staining is conducted according to previously reported protocols¹¹. Briefly, after the Morris water maze test, the brains are dissected and incubated with 4% paraformaldehyde for 1 d. Brain specimens are dehydrated, paraffin-embedded, and sliced into 4-micron slices. Hippocampal sections are dewaxed and rehydrated. Treatment with 3% H₂O₂ is conducted for 10 min to inhibit a cellular peroxidase activity. Sections are incubated overnight at 4°C with primary antibodies to a CD45 antigen and a glial fibrillary acidic protein (GFAP). The sections are rinsed and incubated with the secondary antibody for 30 min at 37°C. The sections are labeled with DAB and analyzed colorimetrically. A mean OD of the area is analyzed using Image Pro 6.0 (Media Cybernetics Inc., MD, USA). The resulting digital images are used for subsequent semi-automated analysis. Color deconvolution analysis is applied. Positive area and OD are determined by measurements on randomly selected microscope fields on each slide. Definition of IHC index: mean OD = optical density × positive area / total area^{12,13}.

### Data analysis and statistics

The results are expressed as mean ± standard deviation. Differences between groups are compared using one-way ANOVA and a Tukey's test. P < 0.05 is considered statistically significant.

### Results

DXMT ameliorates acyclovir-induced weight loss in mice with Aβ oligomers.

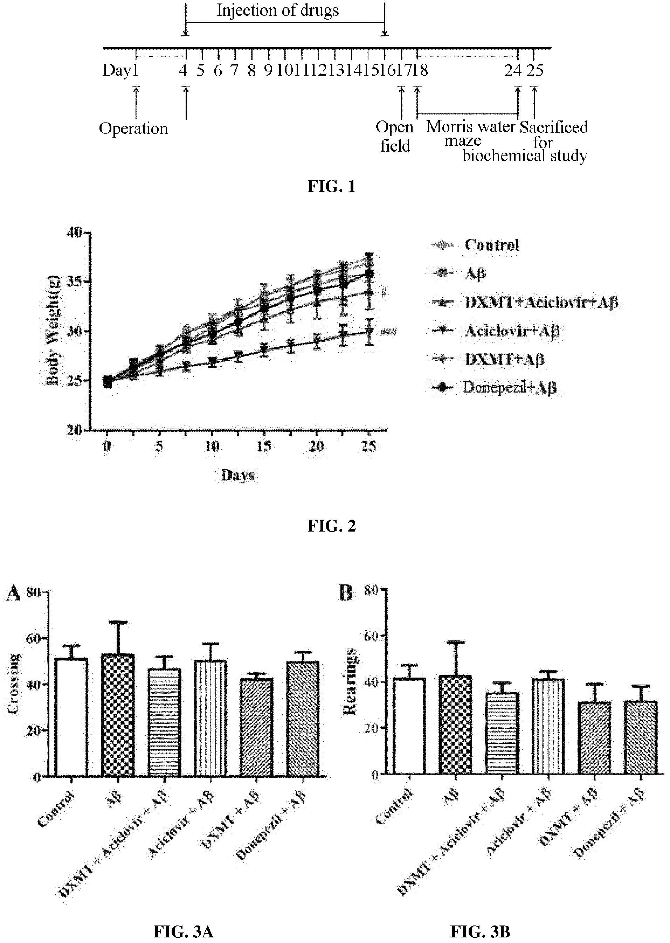

The whole process of the animal experiment is shown in FIG. 1. Briefly, on day 1, the Aβ oligomers are injected into both sides of the mouse hippocampus. The mice are let recover for 3 d. On days 4 to 16, mice are treated differently for 13 d. On day 17, a motor function of the mice is examined by the open field test. On days 18-24, the spatial cognition is tested by the Morris water maze test. On day 25, the mice are sacrificed for biochemical studies.

Digestive disturbance is one of the common adverse effects of acyclovir. Long-term treatment with acyclovir may cause indigestion and malnutrition, leading to weight loss. Therefore, the body weight of the mice is measured every 2 d to 3 d during the experiment. Two-way repeated ANOVA shows that time effect [two-way ANOVA, F (10, 385) = 567.5, P < 0.00], treatment effect [two-way ANOVA, F (5, 385) = 274.8, P < 0.00] and treatment × time interaction [two-way ANOVA, F (50, 385) = 8.4, P < 0.001; FIG. 2] have a significant effect. On the last day of experiment, donepezil treatment does not change the body weight compared with the control group (P > 0.05, FIG. 2). However, acyclovir and acyclovir + DXMT treatments significantly reduce the body weight compared to the control group, suggesting that the acyclovir may lead to the weight loss in mice (p<0.05, FIG. 2). Interestingly, the body weight is significantly higher in the acyclovir + DXMT group than that in the acyclovir group, suggesting that the DXMT may ameliorate acyclovir-induced weight loss in Aβ oligomer-treated mice.

Acyclovir and DXMT have no significant effect on the motor function of mice.

To further investigate whether the acyclovir or DXMT affects the motor function of the animals, the motor activity of the mice is evaluated by the open field test. As shown in FIG. 3, there are no significant differences in the number of standing or crossing lines, indicating that the acyclovir and DXMT treatments do not significantly alter the motor function in mice [the number of standing, one-way ANOVA, f (5, 13) = 0.1182, p > 0.05, FIG. 3a; the number of crossing lines, f(5, 13) = 0.1937, p > 0.05, FIG. 3b].

The combination of acyclovir and DXMT, rather than acyclovir or DXMT used alone, can significantly attenuate Aβ oligomer-induced spatial cognitive impairment.

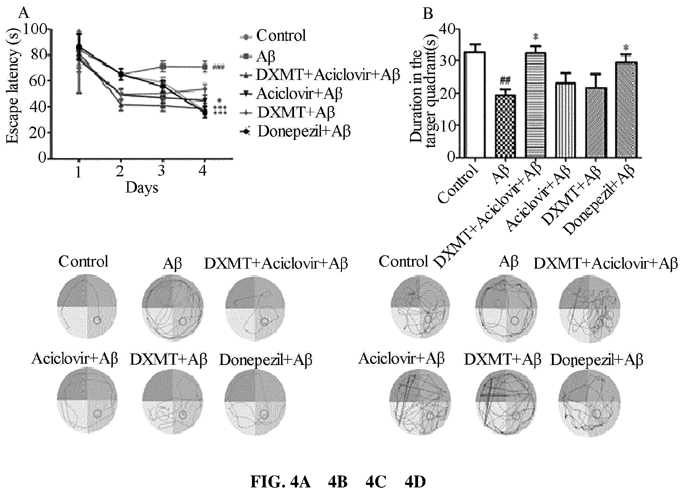

Two-way repeated ANOVA shows that time effect [two-way ANOVA, F (3, 244) = 163.2, P < 0.001, FIG. 4A], treatment effect [two-way ANOVA, F (5, 244) = 27.84, P < 0.001, FIG. 4A], and treatment × time interaction [two-way ANOVA, F (15, 244) = 7.502, P < 0.001, FIG. 4A] have a significant effect on Morris water maze training. On the last day of training, mice in the Aβ group find the hidden platform with a significantly longer time than that in the control group (p<0.001, FIG. 4A). In addition, the time in the acyclovir + DXMT group and the donepezil group is significantly shorter than that in the Aβ group (p<0.001, FIG. 4A). Mice in the acyclovir or DXMT group do not show a better performance compared to the mice in the Aβ group under the same conditions (p>0.05, FIG. 4A). These results suggest that the combination of acyclovir and DXMT, rather than acyclovir or DXMT used alone, can attenuate Aβ oligomer-induced spatial learning impairment in mice.

The trial has also recorded a time that mice are in a target area during the trial. There are significant differences among different groups [one-way ANOVA, F (5, 54) = 5.653, P < 0.01,

FIG. 4B]. Compared with the control group, mice in the Aβ group have a significantly less time in the target area (p<0.01, FIG. 4B). The time in the acyclovir + DXMT group and the donepezil group is significantly longer than that in the Aβ group (p<0.05, FIG. 4B). Mice in the acyclovir or DXMT group do not show a better performance compared to the mice in the Aβ group under the same conditions (p>0.05, FIG. 4B). These results suggest that the combination of acyclovir and DXMT, rather than acyclovir or DXMT used alone, can attenuate Aβ oligomer-induced spatial memory impairment in mice.

Combination of the acyclovir and the DXMT significantly reduces Aβ oligomer-induced overexpression of pro-inflammatory cytokines.

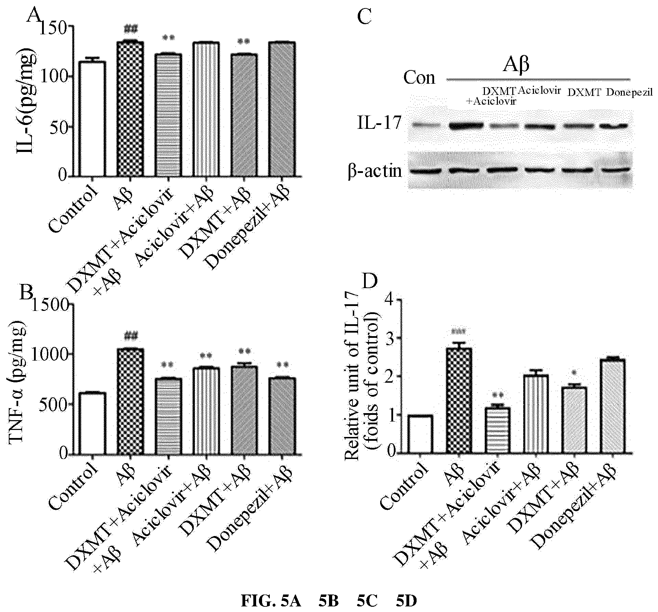

The trail further evaluates the expression of pro-inflammatory cytokines in the mouse hippocampus. The contents of TNF-α and IL-6 in mouse hippocampus are measured by the ELISA. The expressions of TNF-α and IL-6 in the Aβ group are significantly higher than those in the control group (P<0.01, FIGs. 5A and 5B). The acyclovir + DXMT treatment significantly reduces Aβ-induced increases in the TNF-α and IL-6 expression (p<0.01, FIGs. 5A and 5B). Acyclovir alone does not significantly reduce IL-6 production, suggesting that DXMT may play a major anti-inflammatory role in the combination therapy (p>0.05, FIG. 5A). Under the same conditions, the donepezil treatment does not alter the expression of IL-6 compared with the Aβ group (p>0.05, FIG. 5A).

The expression of pro-inflammatory cytokine IL-17 is detected by the Western blotting. The expression of IL-17 in the Aβ group is significantly higher than that in the control group (P<0.01, FIGs. 5C and 5D). The Aβ-induced increase in IL-17 expression is reduced by the DXMT group and the acyclovir + DXMT group (p<0.05, FIGs. 5C and 5D). Under the same conditions, acyclovir cannot reduce the expression of IL-17 in the hippocampus of mice, further supporting that the DXMT may mainly act as an anti-inflammatory drug in the combination therapy (p>0.05, FIG. 5D). Under the same conditions, the donepezil treatment does not alter the expression of IL-17 compared with the Aβ group (p>0.05, FIG. 5D).

Combination of the acyclovir and the DXMT significantly reduces Aβ oligomer-induced activation of microglia and astrocytes.

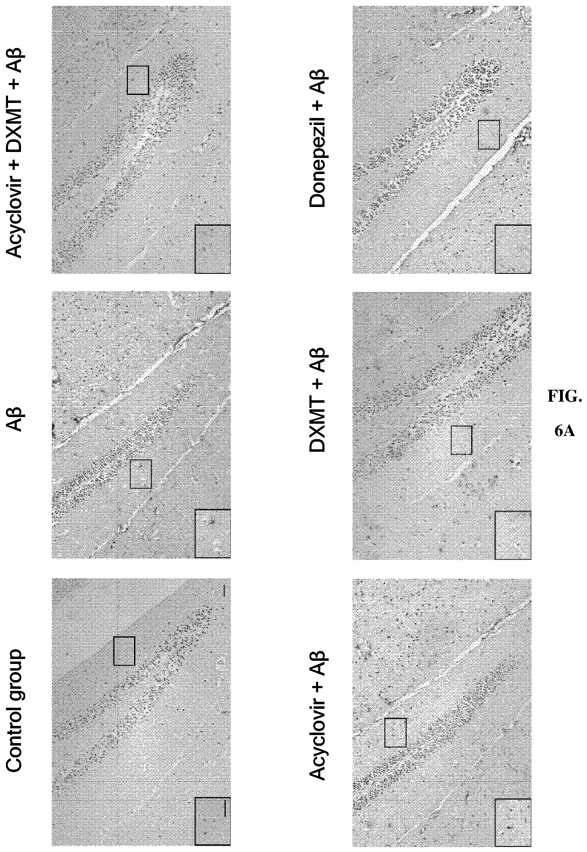

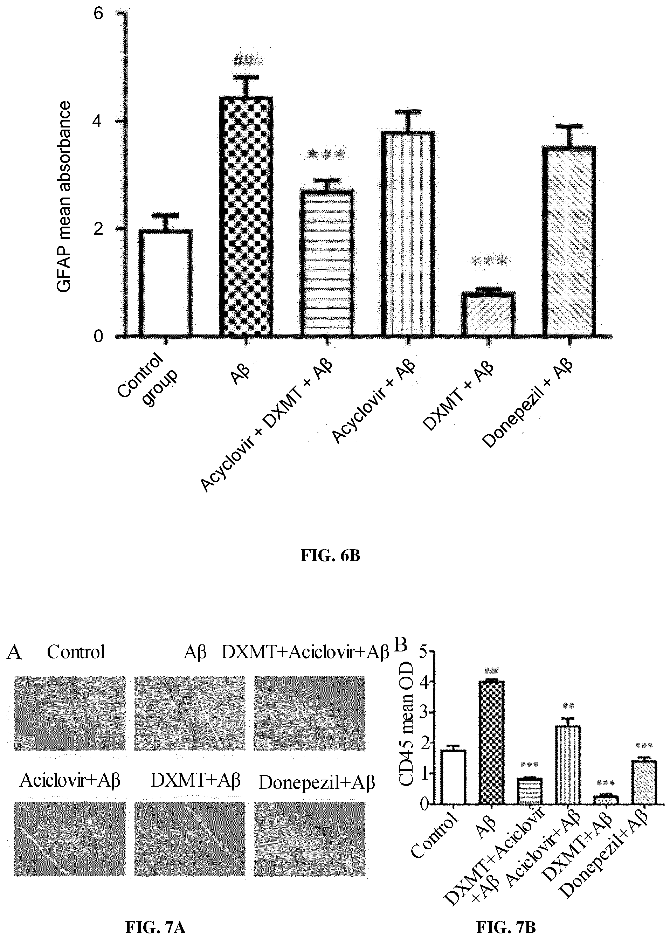

To further investigate whether the combination of acyclovir and DXMT affects Aβ oligomer-induced astrogliosis, IHC staining is conducted for GFAP, a biomarker of astrocytes. The Aβ oligomer significantly increases the mean OD of gfap in the hippocampus (p<0.001, FIG. 6). The acyclovir + DXMT group, acyclovir group and DXMT group significantly reduce the Aβ-induced increase in the mean OD of gfap, suggesting that the combination of acyclovir and DXMT can significantly attenuate Aβ-induced astrogliosis (P<0.001, FIG. 6). Under the same conditions, neither acyclovir nor donepezil treatment can change the mean OD of GFAP compared with the Aβ group (p>0.05, FIG. 6).

Likewise, the Aβ oligomer significantly increases the mean OD of CD45, a biomarker of activated microglia in the hippocampus (p<0.001, FIG. 7). The acyclovir + DXMT group and DXMT group significantly reduce the Aβ-induced increase in CD45 mean OD, indicating that the combination of acyclovir and DXMT attenuates Aβ-induced microglial activation (p <0.001, FIG. 7).

Combination of the acyclovir and the DXMT significantly reduces Aβ-induced decrease in PSD-95 expression.

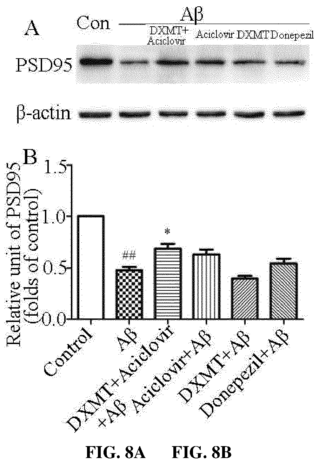

The expression of a postsynaptic protein PSD-95 in the mouse hippocampus is also analyzed by Western blotting. The expression of PSD-95 in the Aβ oligomer group is significantly lower than that in the control group (P<0.01, FIG. 8). In addition, compared with the Aβ group, acyclovir + DXMT group significantly increases the expression of PSD-95 in the hippocampus of mice (P<0.05, FIG. 8). However, the acyclovir, DXMT or donepezil alone does not alter the expression of PSD-95 compared to the Aβ group (p>0.05, FIG. 8). These results suggest that only the combination of acyclovir and DXMT, rather than either acyclovir or DXMT alone, can attenuate the Aβ-induced decrease in PSD-95 expression.

Combination of the acyclovir and the DXMT significantly reduces Aβ oligomer-induced pTau expression.

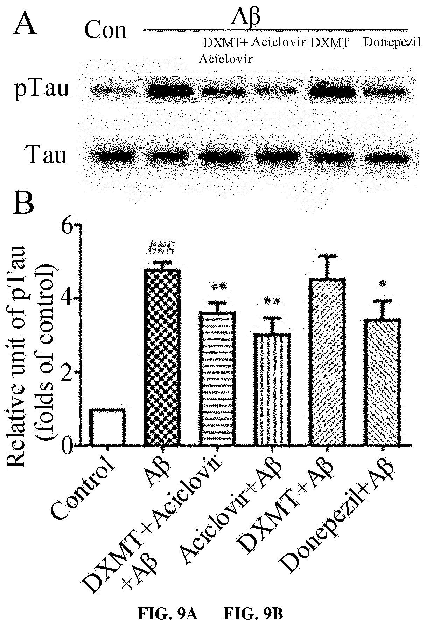

Tau and pTau expressions are also analyzed by Western blotting. The hyperphosphorylation of Tau protein is a hallmark of AD, and the expression of pTau in the Aβ group is significantly higher than that in the control group (P<0.001, FIG. 9). In addition, compared with the Aβ group, the acyclovir + DXMT group attenuates the Aβ-induced increase in pTau expression in the mouse hippocampus (p<0.01, FIG. 9). However, DXMT alone does not alter pTau expression compared to the Aβ group, suggesting that the acyclovir may act as a pTau inhibitor in combination therapy (p>0.05, FIG. 9).

### Discussion

In this study, it is found that the combination of acyclovir and DXMT, rather than acyclovir or DXMT alone, can significantly prevent Aβ oligomer-induced spatial cognitive impairment without affecting the motor function of mice. In addition, the combination of acyclovir and DXMT can significantly reduce Aβ-induced neuroinflammation, while DXMT mainly acts as an anti-inflammatory component.

Acyclovir can penetrate the blood-brain barrier and enter the brain for action. Previous researches suggest that the acyclovir may help prevent AD-related toxicity. HSV-1 is considered a toxin associated with AD pathology¹⁴. Acyclovir treatment reduces HSV-1-induced Aβ accumulation by 70%, and inhibits aberrant tau phosphorylation by nearly 100% in HSV-1-infected cells. Although Aβ-treated mice are free of HSV-1 infection, the acyclovir still has a potential to prevent AD-related neurotoxicity through a similar mechanism. In addition, acyclovir has been reported to inhibit the production of quinolinic acid, a neurotoxic metabolite of tryptophan. Abnormal tryptophan metabolism is widely observed in AD, leading to the formation of quinolinic acid, an agonist of NMDA receptors. The quinolinic acid further increases tau hyperphosphorylation in cortical neurons and causes neurotoxicity. The acyclovir can directly inhibit the activities of indoleamine 2,3-dioxygenase 1 (IDO-1) and tryptophan 2,3-dioxygenase 2 (TDO-2); these two key enzymes are responsible for tryptophan metabolism to prevent quinolinic acid-induced neurotoxicity. Acyclovir is found to significantly attenuate the Aβ-induced increase in pTau expression, which is consistent with previous studies that acyclovir significantly reduced Aβ and pTau accumulation *in vitro*¹⁵*.* It is speculated that acyclovir may inhibit the accumulation of PTAU by inhibiting the activities of IDO-1 and TDO-2. However, in the present disclosure, acyclovir alone does not significantly improve neuroinflammation in mice. Acyclovir has been reported to inhibit the replication of hsv-1 in the brain but not the inflammatory response induced by hsv-1, suggesting that the acyclovir may not be sufficient to produce anti-neuroinflammation effects^{16,17}. These studies are consistent with the present finding that acyclovir does not largely reduce neuroinflammation in Aβ mice.

Glucocorticoids can inhibit inflammation in the brain, improve prognosis, and reduce mortality from neurological diseases. Therefore, it is speculated that the DXMT helps to suppress neuroinflammation in Aβ mice. Acyclovir and DXMT are commonly used clinical drugs. The toxicity, absorption and pharmacokinetics of these two drugs are well studied and there are not too many safety concerns. Previous studies have shown that the combined treatment of acyclovir and glucocorticoids in mice with virus infection of early herpes simplex encephalitis can effectively relieve the neurological symptoms of herpes simplex virus-infected mice. The research results of the present disclosure show that the combination of acyclovir and DXMT can effectively prevent Aβ-induced cognitive impairment and neuroinflammation, further proving effectiveness of the combination of acyclovir and glucocorticoids in treating AD and other neurodegenerative diseases.

The most common side effect of acyclovir is renal insufficiency due to nonspecific inflammation, leading to acute renal failure. DXMT can effectively inhibit the nonspecific inflammation, and can be clinically used for acute renal failure caused by acyclovir. This is another reason to use the combination of acyclovir and DXMT in the studies. Previous studies have shown that acyclovir can cause weight loss, which may be related to renal impairment. In the present study, DXMT is found to improve acyclovir-induced weight loss, suggesting that the DXMT can also reverse acyclovir-induced side effects.

Microglia are macrophages that make up about 10% of all cells in the brain, and constitute a first line of defense against brain damages for the cells²⁰. Previous studies have shown that activation of microglia is associated with AD progression²¹. Activated microglia produce inflammatory cytokines that lead to neuronal damages. In addition, activated astrocytes can participate in neurotoxicity by secreting various inflammatory cytokines, such as IL-6, IL-17, and TNF-α²². Furthermore, astrocytes can interact with microglia, leading to mutual activation²³. Present studies have found that acyclovir can inhibit the activation of microglia, but not astrocytes, which is consistent with previous studies; ganciclovir, an anti-herpes derivative of the acyclovir, has an ability to inhibit activation of microglia but not astrocytes²⁴. DXMT has been reported to inhibit astrocyte activation through a glucocorticoid receptor pathway²⁵. In addition, the DXMT attenuates the microglial response of herpes simplex²⁶. The findings of the present disclosure show that DXMT can inhibit the activation of astrocytes and microglia, which is consistent with existing reports. Therefore, this combination drug can exert both anti-inflammatory and immunoregulation effects.

Synapses may be responsible for memory formation and storage²⁷. PSD-95 is a major scaffolding protein that determines the structural and functional integrity of synapses²⁸. Synaptic loss is considered a major pathological event in AD progression²⁹. In the present study, it is found that the combination of acyclovir and DXMT, rather than the drugs alone, inhibits the loss of PSD-95 to a large extent. This suggests that drug combination may prevent Aβ-induced cognitive impairment by acting on the synapses.

Notably, high levels of glucocorticoids during long-term treatment can create glucocorticoid resistance and impair glucocorticoid receptor function in the brain, exacerbating cognitive impairment in AD progression⁴. However, the response to glucocorticoids depends on several factors, including the duration and intensity of stimulation. Glucocorticoids may act as anti-inflammatory molecules under pathological conditions³⁰. Under physiological conditions, the glucocorticoids may play a pro-inflammatory role. In the present study, DXMT can produce anti-inflammatory effects and reduce Aβ-induced increases in IL-6 and TNF-α expression. It is speculated that this may be because the mice are treated with short-term low concentrations of DXMT. Interestingly, high levels of glucocorticoids during long-term treatment can lead to neurotoxicity by increasing the tryptophan metabolite, quinolinic acid. Therefore, the quinolinic acid inhibitor acyclovir may also counteract the cognitive impairment induced by high levels of glucocorticoids, further supporting advantages of the combination of acyclovir and DXMT in AD treatment⁴.

In summary, the present disclosure finds for the first time that the combination of acyclovir and DXMT may prevent Aβ-induced cognitive impairment by reducing neuroinflammation, synaptic damages, tau hyperphosphorylation, and activation of astrocytes and microglia. Since acyclovir and DXMT are commonly used clinical drugs, this study may provide a new strategy for the clinical treatment of AD as lead or adjuvant drugs.

### BRIEF DESCRIPTION OF THE DRAWINGS

FIG. 1 shows an experimental design of the whole behavioral tests; where briefly, on day 1, Aβ is injected into mouse hippocampus; the mice are let recover for 3 d; on day 4, the mice are given the drug for 13 d; on day 17, a motor function of the mice is examined by the open field test; on days 18-24, the spatial cognition is tested by the Morris water maze test; the body weight is measured every 2 d to 3 d; on day 25, the mice are sacrificed for biochemical studies;
FIG. 2 shows that a body weight of mice was significantly lower in acyclovir and acyclovir + DXMT groups than the control groups; during the experiment, the body weight of mice is measured every 2 d to 3 d; data represents mean ± standard deviation (n = 10); ^{#}p < 0.05 and ^{###}p < 0.001 compared to the control group on day 25 (one-way ANOVA and Tukey's test);
FIG. 3 shows that the motor function of mice was not significantly altered among groups; in an open field experiment, the number of standing and the number of crossing lines in each group are shown in FIG. 3A and FIG. 3B, separately; data is presented as mean ± standard deviation (n = 10);
FIG. 4 shows that a combination of acyclovir and DXMT significantly attenuates Aβ oligomer-induced impairments of spatial cognition as demonstrated by the Morris water maze test; FIG. 4A shows that a latency of the acyclovir group and the acyclovir + DXMT group after training is significantly shorter than that of an Aβ group; FIG. 4B shows that in the trail, a duration of the mice in a target area of the acyclovir + DXMT group is significantly longer than that in a low Aβ group; representative swimming trajectories of mice in the trial are shown in FIG. 4C to FIG. 4D; data represents mean ± SD (n = 10); ^{##}p < 0.01 and ^{###}p < 0.001 compared to the control group; *p < 0.05 and ***p < 0.001 compared to the Aβ group (one-way ANOVA and Tukey's test); FIG. 4C shows a representative swimming trajectory of the mice during a training period; FIG. 4D shows a representative swimming trajectory of the mice during a latency period;
FIG. 5 shows that the combination of acyclovir and DXMT significantly attenuates Aβ oligomer-induced over-expression of pro-inflammatory cytokines; contents of TNF-α in FIG. 5A and IL-6 in FIG. 5B in hippocampal extracts of each group are determined by ELISA, separately; FIG. 5C shows expressions of IL-17 and β-actin in mouse hippocampal extracts detected by Western blotting; a quantitative analysis of an IL-17 level is shown in FIG. 5D; data represents mean ± standard deviation (n = 4 in FIG. 5A and FIG. 5B, and n=3 in FIG. 5D); ^{##}p < 0.01 and ^{###}p < 0.001 compared to the control group; and *p < 0.05 and **p < 0.01 compared to the Aβ group (one-way ANOVA and Tukey's test);
FIG. 6 shows that the combination of acyclovir and DXMT significantly attenuates Aβ oligomer-induced over-activation of astrocytes in the hippocampal region of mice; FIG. 6A shows a typical image of GFAP staining in the hippocampus of each group; a quantitative analysis of a mean OD of the GFAP staining is shown in FIG. 6B; data represents mean ± standard deviation (n = 3); ^{###}p<0.001 for the control group, and ***p<0.001 for the Aβ group (one-way ANOVA and Tukey's test); scale bar is: 30 microns;
FIG. 7 shows that the combination of acyclovir and DXMT significantly attenuates Aβ oligomer-induced over-activation of microglia in the hippocampal region of mice; FIG. 7A shows a typical image of CD45 staining in the hippocampus of each group; a quantitative analysis of a mean OD of the CD45 staining is shown in FIG. 7B; data represents mean ± standard deviation (n = 3); ^{###}p < 0.001 relative to the control group; **p < 0.01 and ***p < 0.001 relative to the Aβ group (one-way ANOVA and Tukey's test); scale bar is: 30 microns;
FIG. 8 shows that the combination of acyclovir and DXMT attenuates Aβ oligomer-induced decrease of PSD-95 expression in the hippocampal region of mice; FIG. 8A shows expressions of PSD-95 and β-actin detected by Western blotting; a quantitative analysis of PSD-95 expression is shown in FIG. 8B; data represents mean ± standard deviation (n = 4); ^{##}p<0.01 for the control group, and *p<0.05 for the Aβ group (one-way ANOVA and Tukey's test);
FIG. 9 shows that the combination of acyclovir and DXMT attenuates Aβ oligomer-induced increase expression of pTau in the hippocampal region of mice; FIG. 9A shows expressions of Tau and pTau determined by Western blotting; a quantitative analysis of pTau expression is shown in FIG. 9B; data represents mean ± SD (n = 4); ^{###}p < 0.001 for the control group, and *p<0.05 and ***p<0.001 for the Aβ group (one-way ANOVA and Tukey's test).

### DETAILED DESCRIPTION OF THE EMBODIMENTS

The present disclosure is further illustrated through the following examples, but the examples are not intended to limit the present disclosure.

### Example 1

### Acyclovir and DXMT granules

100 g of acyclovir and 0.75 g of DXMT, lactose, starch, and low-substituted hydroxypropyl cellulose were sieved through a 60-mesh sieve, separately, weighed according to a formula, and mixed in a mixer for 30 min; an appropriate amount of a binder was added to prepare a soft material, granulated by 20 mesh, dried, and sieved by 18 mesh to obtain a mixed powder; a prescribed amount of magnesium stearate was added to the granulated mixed powder for mixing, and a resulting product was divided to obtain the granules.

### Example 2

### Acyclovir and DXMT capsules

100 g of acyclovir and 0.75 g of DXMT, lactose, starch, and low-substituted hydroxypropyl cellulose were sieved through a 60-mesh sieve, separately, weighed according to a formula, and mixed in a mixer for 30 min; an appropriate amount of a binder was added to prepare a soft material, granulated by 20 mesh, dried, and sieved by 18 mesh to obtain a mixed powder; a prescribed amount of magnesium stearate was added to the granulated mixed powder for mixing, and a resulting product was placed in a capsule filling machine for filling into the capsules.

### Example 3

### Acyclovir and DXMT tablets

100 g of acyclovir and 0.75 g of DXMT, lactose, starch, and low-substituted hydroxypropyl cellulose were sieved through a 60-mesh sieve, separately, weighed according to a formula, and mixed in a mixer for 30 min; an appropriate amount of a binder was added to prepare a soft material, granulated by 20 mesh, dried, and sieved by 18 mesh to obtain a mixed powder; a prescribed amount of magnesium stearate was added to the granulated mixed powder for mixing evenly, and an appropriate amount of a resulting product was sampled for content measurement; a tablet weight was calculated according to a measured content, tabletting was conducted, followed by film coating on obtained tablets.

### Literature Cited

1. Müller, A. C. & Santy, D. Acyclovir inhibits rat liver tryptophan-2,3-dioxygenase and induces a concomitant rise in brain serotonin and 5-hydroxyindole acetic acid levels. Metabolic Brain Disease 23, 351-360 (2008).
2. Itzhaki, R. F. Herpes simplex virus type 1 and Alzheimer's disease: increasing evidence for a major role of the virus. Frontiers in Aging Neuroscience 6, 202 (2014).
3. Li, L., Ma, P., Wei, J., Qian, K. & Tao, L. LC-ESI-MS method for the determination of DXMT acetate in skin of nude mouse. Journal of Chromatography B Analytical Technologies in the Biomedical & Life Sciences 933, 44-49(2013).
4. Vyas, S. et al. Chronic Stress and Glucocorticoids: From Neuronal Plasticity to Neurodegeneration. Neural Plast 2016,6391686,doi: 10.1155/2016/6391686 (2016).
5. Joshi, Y. B., Chu, J. & Pratico, D. Stress hormone leads to memory deficits and altered tau phosphorylation in a model of Alzheimer's disease. J Alzheimers Dis 31,167-176,doi: 10.3233/JAD-2012-120328 (2012).
6. Chen, Z. H. et al. Effect of DXMT and acyclovir on prognosis and expression of IL-2 in mice with herpes simplex virus encephalitis. Journal of Clinical Pediatrics (2012).
7. Lin, J. et al. Fucoxanthin, a Marine Carotenoid, Attenuates β-Amyloid Oligomer-Induced Neurotoxicity Possibly via Regulating the PI3K/Akt and the ERK Pathways in SH-SY5Y Cells. Oxidative Medicine & Cellular Longevity 2017, 6792543 (2017).
8. Kim, H. Y., Lee, D. K., Chung, B. R., Kim, H. V. & Kim, Y. S. Intracerebroventricular Injection of Amyloid-β Peptides in Normal Mice to Acutely Induce Alzheimer-like Cognitive Deficits. Journal of Visualized Experiments Jove 2016 (2016).
9. Wu, X. et al. Curcumin attenuates surgery-induced cognitive dysfunction in aged mice. Metabolic Brain Disease 32, 1-10 (2017).
10. Huang, L. et al. Sunitinib, a clinically used anti-cancer drug, is a potent AChE inhibitor and attenuates cognitive impairments in mice. Acs Chemical Neuroscience 7,1047 (2016).
11. Yu, J.et al. Fucoxanthin prevents H2O2-induced neuronal apoptosis via concurrently activating the PI3-K/Akt cascade and inhibiting the ERK pathway. Food & Nutrition Research 61, 1304678 (2017).
12. Sushmita, C. et al. Quantitative immunohistochemical analysis reveals association between sodium iodide symporter and estrogen receptor expression in breast cancer. Plos One 8, e54055 (2013).
13. Gu, K. et al. A natural process of cirrhosis resolution and deceleration of liver regeneration after thioacetamide withdrawal in a rat model. Molecular Biology Reports 38,1687-1696 (2011).
14. Harris, S. A. & Harris, E. A. Molecular Mechanisms for Herpes Simplex Virus Type 1 Pathogenesis in Alzheimer's Disease. Front Aging Neurosci 10, 48, doi: 10.3389/fnagi. 2018.00048 (2018).
15. Wozniak, M. A., Frost, A. L., Preston, C. M. & Itzhaki, R. F. Antivirals reduce the formation of key Alzheimer's Disease molecules in cell cultures acutely infected with herpes simplex virus type 1. Plos One 6, e25152 (2011).
16. Lundberg, P., Openshaw, H., Wang, M., Yang, H. J. & Cantin, E. Effects of CXCR3 signaling on development of fatal encephalitis and corneal and periocular skin disease in HSV-infected mice are mouse-strain dependent. Invest Ophthalmol Vis Sci 48, 4162-4170,doi: 10.1167/iovs. 07-0261 (2007).
17. Golub, M. S. et al. Effects of Acyclovir and IVIG on Behavioral Outcomes after HSV1 CNS Infection. Behavioural Neurology, 2017, (2017-11-19) 2017, 1-14 (2017).
18. Seedat, A. & Winnett, G. Acyclovir-induced acute renal failure and the importance of an expanding waist line. BMJ Case Rep 2012,doi: 10.1136/bcr-2012-006264 (2012).
19. Lu, H., Han, Y. J., Xu, J. D., Xing, W. M. & Chen, J. Proteomic characterization of acyclovir-induced nephrotoxicity in a mouse model. PLoS One 9, e103185, doi: 10.1371/journal. pone. 0103185 (2014).
20. Lawson, L. J., Perry, V. H., Dri, P.,. & Gordon, S.,. Heterogeneity in the distribution and morphology of microglia in the normal adult mouse brain. Neuroscience 39, 151-170 (1990).
21. Solito, E. & Sastre, M. Microglia Function in Alzheimer's Disease. Front Pharmacol 3,14 (2012)
22. Deng, Y. et al. Astrocyte-Derived Proinflammatory Cytokines Induce Hypomyelination in the Periventricular White Matter in the Hypoxic Neonatal Brain. Plos One 9, e87420(2014).
23. Hailer, N. P., Glomsda, B. & Blaheta, R. A. Astrocytic factors down-regulate the expression of major histocompatibility complex-class-II and intercellular adhesion molecule-1 on human monocytes. Neuroscience Letters 298,33-36 (2001).
24. Ding, Z. et al. Antiviral drug ganciclovir is a potent inhibitor of microglial proliferation and neuroinflammation. J Exp Med 211, 189-198, doi: 10.1084/jem. 20120696 (2014).
25. Crossin, K. L., Tai, M. H., Krushel, L. A., Mauro, V. P. & Edelman, G. M. Glucocorticoid Receptor Pathways are Involved in the Inhibition of Astrocyte Proliferation. Proceedings of the National Academy of Sciences of the United States of America 94, 2687-2692 (1997).
26. Kozai, T. D. Y., Jaquins-Gerstl, A. S., Vazquez, A. L., Michael, A. C. & Cui, X. T. DXMT retrodialysis attenuates microglial response to implanted probes in vivo. Biomaterials 87, 157-169, doi: 10.1016/j. biomaterials. 2016.02.013 (2016).
27. Sjostrom, P. J., Rancz, E. A., Roth, A. & Hausser, M. Dendritic excitability and synaptic plasticity. Physiol Rev 88, 769-840, doi: 10.1152/physrev. 00016.2007 (2008).
28. Shao, C. Y., Mirra, S. S., Sait, H. B., Sacktor, T. C. & Sigurdsson, E. M. Postsynaptic degeneration as revealed by PSD-95 reduction occurs after advanced Abeta and tau pathology in transgenic mouse models of Alzheimer's Disease. Acta Neuropathol 122, 285-292, doi: 10.1007/s00401-011-0843-x (2011).
29. Rogers, J. T. et al. Subacute ibuprofen treatment rescues the synaptic and cognitive deficits in advanced-aged mice. Neurobiology of Aging 53,112-121 (2017).
30. Cruz-Topete, D. & Cidlowski, J. A. One hormone, two actions: anti-and pro-inflammatory effects of glucocorticoids. Neuroimmunomodulation 22, 20-32, doi: 10.1159/000362724 (2015).

## Claims

1. Combination of acyclovir and dexamethasone (DXMT) for use in treating Alzheimer's disease (AD).

2. The combination of acyclovir and dexamethasone (DXMT) for use according to claim 1, wherein the drug is capable of alleviating a digestive problem possibly caused by the acyclovir.

3. The combination of acyclovir and dexamethasone (DXMT) for use according to claim 1, wherein the acyclovir and the DXMT are prepared into a compound pharmaceutical composition.

4. The combination of acyclovir and dexamethasone (DXMT) for use according to claim 3, wherein a weight ratio of the acyclovir to the DXMT in the compound pharmaceutical composition is (3000-200): (10-0.1).

5. The combination of acyclovir and dexamethasone (DXMT) for use according to claim 4, wherein the weight ratio is 500:(10-0.1).

6. The combination of acyclovir and dexamethasone (DXMT) for use according to claim 4, wherein the weight ratio is 500:(1.5-2).

7. The combination of acyclovir and dexamethasone (DXMT) for use according to any one of claims 1 to 6, wherein an administration route of the drug is selected from the group consisting of a gastrointestinal administration, an intravenous administration, an intramuscular administration, a subcutaneous administration, an oral mucosa administration, a sublingual administration, an oral spray administration, and a nasal spray administration.

8. The combination of acyclovir and dexamethasone (DXMT) for use according to any one of claims 1 to 6, wherein the drug is prepared into an oral preparation selected from the group consisting of a tablet, a capsule, a granule, an oral liquid, and a spray.

9. The combination of acyclovir and dexamethasone (DXMT) for use according to claim 8, wherein the oral preparation comprises 500 mg of the acyclovir and 1.5 mg of the DXMT.

10. The combination of acyclovir and dexamethasone (DXMT) for use according to claim 1, wherein the drug is capable of being combined with a target therapy for senile dementia and the AD based on immune balance.

11. The combination of acyclovir and dexamethasone (DXMT) for use according to claim 1, wherein the drug is used in pregnant women, newborn infants, infants, children, adults, and elderly, especially in fatal brain infections caused by a human herpes simplex virus and near-derived varicella virus infections, with post-treatment immune memory protection.

## Patentansprüche

1. Kombination von Aciclovir und Dexamethason (DXMT) zur Verwendung bei der Behandlung der Alzheimer-Krankheit (AD).

2. Kombination von Aciclovir und Dexamethason (DXMT) zur Verwendung nach Anspruch 1, wobei das Arzneimittel in der Lage ist, ein möglicherweise durch Aciclovir verursachtes Verdauungsproblem zu lindern.

3. Kombination von Aciclovir und Dexamethason (DXMT) zur Verwendung nach Anspruch 1, wobei das Aciclovir und das DXMT zu einer zusammengesetzten pharmazeutischen Zusammensetzung verarbeitet werden.

4. Kombination von Aciclovir und Dexamethason (DXMT) zur Verwendung nach Anspruch 3, wobei ein Gewichtsverhältnis von Aciclovir zu DXMT in der zusammengesetzten pharmazeutischen Zusammensetzung (3000-200): (10-0,1) ist.

5. Kombination von Aciclovir und Dexamethason (DXMT) zur Verwendung nach Anspruch 4, wobei das Gewichtsverhältnis 500:(10-0,1) ist.

6. Kombination von Aciclovir und Dexamethason (DXMT) zur Verwendung nach Anspruch 4, wobei das Gewichtsverhältnis 500: (1,5-2) ist.

7. Kombination von Aciclovir und Dexamethason (DXMT) zur Verwendung nach einem der Ansprüche 1 bis 6, wobei ein Verabreichungsweg des Arzneimittels aus der Gruppe, bestehend aus einer Magen-Darm-Verabreichung, einer intravenösen Verabreichung, einer intramuskulären Verabreichung, einer subkutanen Verabreichung, einer Verabreichung über die Mundschleimhaut, einer sublingualen Verabreichung, einer Verabreichung als Mundspray und einer Verabreichung als Nasenspray, ausgewählt ist.

8. Kombination von Aciclovir und Dexamethason (DXMT) zur Verwendung nach einem der Ansprüche 1 bis 6, wobei das Arzneimittel als orale Zubereitung, ausgewählt aus der Gruppe, bestehend aus einer Tablette, einer Kapsel, einem Granulat, einer oralen Flüssigkeit und einem Spray, hergestellt wird.

9. Kombination von Acyclovir und Dexamethason (DXMT) zur Verwendung nach Anspruch 8, wobei die orale Zubereitung 500 mg Acyclovir und 1,5 mg DXMT umfasst.

10. Kombination von Aciclovir und Dexamethason (DXMT) zur Verwendung nach Anspruch 1, wobei das Arzneimittel in der Lage ist, mit einer Zieltherapie für Altersdemenz und AD basierend auf dem immunologischen Gleichgewicht kombiniert zu werden.

11. Kombination von Aciclovir und Dexamethason (DXMT) zur Verwendung nach Anspruch 1, wobei das Arzneimittel bei schwangeren Frauen, Neugeborenen, Säuglingen, Kindern, Erwachsenen und älteren Menschen insbesondere bei durch ein humanes Herpes-simplex-Virus und eng verwandte Varizella-Virus-Infektionen hervorgerufenen tödlichen Hirninfektionen mit Nachbehandlungsschutz durch immunologisches Gedächtnis verwendet wird.

## Revendications

1. Combinaison d'acyclovir et de dexaméthasone (DXMT) pour utilisation dans le traitement de la maladie d'Alzheimer (AD).

2. Combinaison d'acyclovir et de dexaméthasone (DXMT) pour utilisation selon la revendication 1, dans laquelle le médicament est capable de soulager un problème digestif éventuellement causé par l'acyclovir.

3. Combinaison d'acyclovir et de dexaméthasone (DXMT) pour utilisation selon la revendication 1, dans laquelle l'acyclovir et la DXMT sont préparés en une composition pharmaceutique composée.

4. Combinaison d'acyclovir et de dexaméthasone (DXMT) pour utilisation selon la revendication 3, dans laquelle un rapport en poids de l'acyclovir par rapport à la DXMT dans la composition pharmaceutique composée est de (3000-200): (10-0,1).

5. Combinaison d'acyclovir et de dexaméthasone (DXMT) pour utilisation selon la revendication 4, dans laquelle le rapport en poids est de 500:(10-0,1).

6. Combinaison d'acyclovir et de dexaméthasone (DXMT) pour utilisation selon la revendication 4, dans laquelle le rapport en poids est de 500: (1,5-2).

7. Combinaison d'acyclovir et de dexaméthasone (DXMT) pour utilisation selon l'une quelconque des revendications 1 à 6, dans laquelle une voie d'administration du médicament est choisie parmi une administration gastro-intestinale, une administration intraveineuse, une administration intramusculaire, une administration sous-cutanée, une administration par muqueuse orale, une administration sublinguale, une administration par pulvérisation orale et une administration par pulvérisation nasale.

8. Combinaison d'acyclovir et de dexaméthasone (DXMT) pour utilisation selon l'une quelconque des revendications 1 à 6, dans laquelle le médicament est préparé en une préparation orale choisie parmi un comprimé, une capsule, un granulé, un liquide oral et un spray.

9. Combinaison d'acyclovir et de dexaméthasone (DXMT) pour utilisation selon la revendication 8, dans laquelle la préparation orale comprend 500 mg d'acyclovir et 1,5 mg de DXMT.

10. Combinaison d'acyclovir et de dexaméthasone (DXMT) pour utilisation selon la revendication 1, dans laquelle le médicament est capable d'être combiné avec une thérapie ciblée pour la démence sénile et la AD sur la base de l'équilibre immunitaire.

11. Combinaison d'acyclovir et de dexaméthasone (DXMT) pour utilisation selon la revendication 1, dans laquelle le médicament est utilisé chez les femmes enceintes, les nourrissons nouveau-nés, les nourrissons, les enfants, les adultes et les personnes âgées, en particulier dans les infections cérébrales fatales causées par un virus de l'herpès simplex humain et les infections par le virus de la varicelle dérivé proche, avec protection post-traitement de la mémoire immunitaire.
